(19) Europäisches Patentamt
European Patent Office
Office européen des brevets

(11) Publication number: 0 141 797
B1

(12) EUROPEAN PATENT SPECIFICATION

(45) Date of publication of patent specification: 16.08.89

(51) Int. Cl.$^4$: B 01 J 27/057, C 07 C 120/14

(21) Application number: 84870146.2

(22) Date of filing: 24.10.84

# (54) Catalysts for the oxidation and ammoxidation of olefins and their use making acrylonitrile.

(30) Priority: 24.10.83 US 545025
24.10.83 US 545026

(43) Date of publication of application:
15.05.85 Bulletin 85/20

(45) Publication of the grant of the patent:
16.08.89 Bulletin 89/33

(84) Designated Contracting States:
BE CH DE FR GB IT LI NL SE

(56) References cited:
EP-A-0 000 835
EP-A-0 076 678
EP-A-0 089 118
DE-A-3 226 204
US-A-4 083 804

(73) Proprietor: Monsanto Company
Patent Department 800 North Lindbergh Boulevard
St. Louis Missouri 63167-7020 (US)

(72) Inventor: Li, Tao Ping
293 Heather Crest Drive
Chesterfield Missouri 63017 (US)

(74) Representative: McLean, Peter et al
Monsanto Europe S.A. Patent Department
Avenue de Tervuren 270-272 Letter Box No 1
B-1150 Brussels (BE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 141 797 B1

## Description

A. Field of the invention

This invention relates to oxidation and/or ammoxidation catalysts containing the elements iron, antimony, copper, tellurium, molybdenum, and oxygen, optionally tungsten and vanadium, to a method of preparing such catalysts, and the use of such catalysts in ammoxidation reactions.

It is well known that olefins can be oxidized to oxygenated hydrocarbons such as unsaturated aldehydes and acids, for example, acrolein and methacrolein, and acrylic acid and methacrylic acid. It is also well known that olefins can be ammoxidized to unsaturated nitriles such as acrylonitrile and methacrylonitrile. The value of such oxygenated hydrocarbons and unsaturated nitriles is generally well recognized, with acrylonitrile being among the most valuable monomers available to the polymer industry for producing useful polymeric products. The catalysts of this invention are useful in such oxidation and ammoxidation reactions.

B. Description of the prior art

Various catalytic processes are known for the oxidation and/or ammoxidation of olefins. Such processes commonly react an olefin or an olefin-ammonia mixture with oxygen in the vapor phase in the presence of a catalyst. For the production of acrolein and acrylonitrile, propylene is the generally used olefin reactant, and for the production of methacrolein and methacrylonitrile, isobutylene is the generally used olefin reactant.

Many catalysts are disclosed as suitable in the oxidation and ammoxidation of olefins. One such catalyst is described in U.S. Patents 3,988,359, 4,083,804 and 4,208, 303. This catalyst is represented by the empirical formula:

$$Fe_aSb_bMe_cTe_dQ_eR_fO_g$$

wherein Me is at least one element selected from the group consisting of V, Mo, and W; Q is at least one element selected from the group consisting of Cu, Mg, Zn, La, Ce, Al, Cr, Mn, Co, Ni, Bi, and Sn; and R is at least one element selected from the group consisting of P and B; a, b, c, d, e, f, and g each represents atomic ratios, and when a is 10, b is 15 to 60, c is 1 to 10, d is 0.5 to 10, e is 0.1 to 10, f is 0 to 5 and g represents the number of oxygen atoms corresponding to the oxides resulting from the combination of the above active components. The Me and Te components reportedly are substantially dissolved in an iron-antimony oxide compound ($FeSbO_4$) to form a solid solution.

Although the yield and selectivity of the above-described catalyst are generally satisfactory, it nevertheless suffers from serious drawbacks, principle among which is the loss of Te during extended use. This loss of Te results in a decrease in activity and selectivity over extended periods of use, thereby necessitating costly and involved regeneration of the catalyst via replacement of the Te. Accordingly, the economics in the manufacture of nitriles (for example, acrylonitrile) from olefins (for example, propylene) is severely impacted. And since it is well known that the economics of acrylonitrile manufacture dictate increasingly higher yields and selectivity of conversion of the reactants to acrylonitrile in order to minimize the difficulties attending the purification of the product and handling of large recycle streams, a catalyst which maintains its high activity and selectivity over an extended period of use would be a decided advance in the state of the art.

Summary of the invention

It is the object of this invention to provide a stabilized catalyst composition useful in the preparation of unsaturated nitriles by ammoxidation of olefins, characterized by maintaining high activity and selectivity over an extended period of use.

Another object is to provide a catalyst composition which retains its Te under extended use conditions.

Yet another object is to provide a catalyst which is useful for oxidation of olefins to the corresponding unsaturated aldehyde.

A further object of this invention is to provide a process for manufacture of such catalyst.

It is also an object of this invention to provide a process for the production of acrylonitrile by employing a stabilized catalyst composition characterized by sustained high activity and selectivity over an extended period of use.

To achieve these and other objects which will become apparent from the accompanying description and claims, a mixed oxide catalyst is provided comprising the following elements in the atomic ratios indicated:

$$Fe_aSb_bCu_cTe_dMo_eW_fMe_gO_x$$

Where Me is a member of the group consisting of V, B, P, Mg, Sn, Zn, Mn, and mixtures thereof wherein a is 5 to 10, b is 10 to 30, c is 1 to 5, d is 0.5 to 5, e is 0.1 to 5, f is not greater than 5, g is 0 to 0.5 and x is a number taken to satisfy the valence requirements of the other elements present. According to the present invention, such a catalyst is prepared by forming a mixture containing iron, antimony and copper components, a

preformed tellurium-containing component selected from the group consisting of tellurium molybdate, tellurium tungstate, and mixtures thereof, and a complementary metal-containing component, the metal constituent of which is selected from molybdenum and tungsten and mixtures thereof, with the proviso that when the preformed tellurium-containing component is tellurium molybdate, the metal constituent of the complementary metal-containing component is tungsten and when the preformed tellurium-containing component is tellurium tungstate, the metal constituent of the complementary metal-containing component is molybdenum. Optionally, a vanadium component, in amounts sufficient to satisfy the elemental atomic ratio requirements, and a tungsten component if the complementary metal-containing component does not contain tungsten may be included.

The mixture is formed into particles which are dried and calcined.

Description of the preferred embodiments

In accordance with this invention, a catalyst useful for the oxidation and ammoxidation of olefins is a mixed oxide comprising the following elements in the atomic ratios specified:

$$Fe_aSb_bCu_cTe_dMo_eW_fMe_gO_x$$

where Me is a member of the group consisting of V, B, P, Mg, Sn, Zn, Mn, and mixtures thereof wherein a is 5 to 10, b is 10 to 30, c is 1 to 5, d is 0.5 to 5, e is 0.1 to 5, f is 0 to 5, g is 0 to 0.5, and x is a number taken to satisfy the valence requirements of the other elements present. The catalyst is prepared by forming a mixture containing iron, antimony and copper components and a preformed tellurium-containing component selected from the group consisting of tellurium molybdate, tellurium tungstate, and mixtures thereof, and a complementary metal-containing component, the metal constituent of which is selected from molybdenum, tungsten and mixtures thereof, with the proviso that when the preformed tellurium-containing component is tellurium molybdate, the metal constituent of the complementary metal-containing component is tungsten and when the preformed tellurium- containing component is tellurium tungstate, the metal constituent of the complementary metal-containing component is molybdenum. Optionally, a vanadium component, in amounts sufficient to satisfy the elemental atomic ratio requirements and a tungsten component, if the complementary metal-containing component does not include tungsten, may be included. The mixture is formed into particles, which are dried and calcined. The catalyst is particularly useful during extended periods of use in that it effectively retains its tellurium (Te) by virtue of the Te having been provided initially in the form of a preformed tellurium-containing component selected from the group consisting of tellurium molybdate, tellurium tungstate, and mixtures thereof. As a result, the catalyst is characterized by maintaining high activity and selectivity over such extended periods of use. In another aspect, this invention is directed to a process employing such catalysts.

The term "tellurium molybdate" as used herein means a chemical compound of tellurium, molybdenum, and oxygen. Such compounds may be formed by the interaction of tellurium compounds in which tellurium acts as a cation with salts of molybdic acids such as ammonium molybdates, or by a solid state reaction between tellurium oxide and molybdenum oxide.

Likewise, as used herein, the term "tellurium tungstate" means a chemical compound of tellurium, tungsten, and oxygen. Such compounds may be formed in a manner similar to that previously described for the tellurium molybdate except that salts of tungstic acid such as ammonium tungstates, for example, are employed in place of the salts of molybdic acids, and tungsten oxide is used to replace molybdenum oxide.

When the tellurium-containing component of the catalyst according to this invention is tellurium molybdate, the component may contain oxides of tellurium and/or molybdenum in addition to tellurium molybdate compounds. In a similar manner the tellurium tungstate component may contain oxides of tellurium and/or tungsten. It is preferred, however, to have the tellurium-containing component substantially free of oxides of tellurium molybdenum and/or tungsten. In this manner, loss of tellurium from the catalysts under extended use conditions is substantially reduced or eliminated altogether, thereby permitting long-term and extended use thereof without a substantial decrease in catalyst activity and selectivity.

The tellurium-containing component of the catalyst according to this invention may be prepared by forming an aqueous solution of metallic tellurium in nitric acid, forming an aqueous solution of a molybdenum- or tungsten-containing salt, oxide, or hydroxide, combining these two solutions, and recovering the precipitated tellurium-containing component by filtration or decantation. Accordingly, suitable tellurium component-forming tellurium compounds include, but are not limited to, elemental tellurium when used as a solution with nitric acid, tellurium dioxide, and tellurium halides when hydrolized. Suitable tellurium component-forming molybdenum compounds include molybdenum trioxide, molybdic acid, molybdenum halides, and ammonium molybdates, such as, for example, diammonium molybdate and ammonium paramolybdate. Similarly, suitable tellurium component-forming tungsten compounds include tungsten trioxide tungstic acid, tungsten halides, and ammonium tungstates.

Exemplary of suitable antimony compounds for the antimony component are antimony trioxide, antimony tetroxide, antimony pentoxide, and antimonic acid.

Compounds which form an oxide of antimony after chemical reaction or calcination are also suitable,

for example, metallic antimony, antimony hydroxides, and antimony halides, such as antimony trichloride, antimony tribromide, and antimony pentachloride.

Suitable iron compounds for the iron component can be selected from many types, for example, ferrous oxide, ferric oxide, and ferroferric oxide ($Fe_3O_4$). Compounds which are finally stabilized as an iron oxide after chemical treatment or calcining treatment can also be used. Such compounds include, for example, inorganic iron salts such as iron nitrate, iron sulfate, and iron chloride, or organic acid iron salts such as iron acetate and iron oxalate. Also, iron hydroxide and a solution of metallic iron in nitric acid can be used.

Suitable copper compounds for the copper component include cupric oxide, cupric nitrate, cupric acetate, cupric oxalate, and cupric hydroxide obtained by the hydrolysis of cupric chloride.

The catalysts of this invention, as previously noted, contain a tellurium-containing component selected from the group consisting of tellurium molybdate, tellurium tungstate, and mixtures thereof. When the tellurium-containing component is tellurium molybdate, a tungsten component must be provided. This requirement is readily satisfied by the complementary metal-containing component of the catalyst (which is otherwise selected from the group consisting of molybdenum, tungsten, and mixtures thereof) by selecting tungsten as the metal constituent thereof. Tungsten as the metal constituent is preferably provided as a metal tungstate, for example, copper tungstate or tellurium tungstate. However, tungsten compounds described hereinabove as suitable for use in preparing tellurium tungstate are also suitable.

Similarly, when the tellurium-containing compound is tellurium tungstate, a molybdenum component must be provided. This requirement is satisfied as described above for the tungsten component by selecting molybdenum as the metal constituent of the metal-containing component. Suitable molybdenum compounds include metal molybdates, for example, tellurium molybdate or copper molybdate and those compounds previously described as suitable for use in preparing tellurium molybdate.

When the tellurium-containing component is a mixture of tellurium molybdate and tellurium tungstate, a mixture of molybdenum and tungsten as the metal of the complementary metal-containing constituent may be employed in amounts sufficient to satisfy the elemental atomic ratio requirements of the catalyst.

The catalyst of this invention optionally contains a vanadium component. The vanadium component is preferably provided as vanadium antimonate. However, other vanadium compounds such as vanadium pentoxide, ammonium metavanadate, vanadyl oxalate, and vanadium halides are also suitable.

As used herein, the term "vanadium antimonate" is employed to designate complex mixtures of vanadium, antimony and oxygen. Compounds which may be formed by the interaction of antimony compounds and vanadium compounds are intended to be included within the term "vanadium antimonate" as used herein. The vanadium antimonate" component of these catalysts may contain free oxides of antimony and/or vanadium.

The optional vanadium antimonate component may be a compound which is formed under conditions of calcination in air at elevated temperatures. However, as used herein, the term vanadium antimonate also includes (uncalcined) precursors of true vanadium antimonate compounds. Such precursors are prepared, for example, by preparing an aqueous slurry of a vanadium compound and an antimony compound. The slurry is boiled until the contained solids turn greenish-brown, indicating the partial interaction or a vanadium antimonate compound, the nature of which is not fully understood. The vanadium antimonate precursor may, if desired, be calcined to form a true vanadium antimonate compound.

The vanadium antimonate component of these catalysts may be formed by adding a vanadium-containing compound to an aqueous slurry of a suitable antimony-containing compound, and boiling the resultant slurry. In a preferred preparation, the vanadium and antimony compounds, respectively, are vanadium pentoxide ($V_2O_5$) and antimony trioxide ($Sb_2O_3$). The slurry is boiled until the contained solids turn greenish-brown, indicating the interaction or partial reaction of the two metal oxides to form a precursor of vanadium antimonate. In a still more preferred preparation procedure, a vanadyl oxalate solution is prepared by adding oxalic acid slowly to an aqueous slurry of ammonium metavanadate at about 60°C until evolution of carbon dioxide ($CO_2$) ceases. To the clear blue solution of vanadyl oxalate is added the desired amount of antimony trioxide and the resulting slurry is dried to form the vanadium antimonate precursor. In any case, a particularly preferred embodiment of this invention, the greenish-brown vanadium antimonate precursor is dried and calcined for 0.5 to 24 hours at a temperature of about 500°C to about 800°C preferably about 550°C to about 750°C to form a grayish-black vanadium antimonate compound.

As indicated above, wherever the complementary metal-containing component does not contain tungsten, or where the amount of tungsten is less than desired, a tungsten component may be added in the atomic ratio range indicated or to bring the total amount to within the atomic ratio range indicated. In such cases the tungsten component may be any non-interfering tungsten compound.

Following the preparation of the tellurium-containing components of the catalyst, these components, along with the appropriate metal-containing component and, optionally, the vanadium and tungsten components, are combined in the desired proportions in an aqueous slurry and well mixed. At this point, a suitable catalyst support may be added, if desired, as described hereinbelow.

The slurry, after intimate mixing, is then heated to remove the bulk of the aqueous phase. The concentrated slurry contains a certain amount of water and it is desirable to remove this water by some form of drying process to form a dry catalyst precursor. This can take the form of a simple oven drying

process in which the water-containing solid phase is subjected to a temperature that is sufficiently high to vaporize the water and completely dry the solid phase.

An alternate drying process which may be employed is the so-called spray-drying process in which water-containing solid phase particles are sprayed into contact with hot gas (usually air) so as to vaporize the water. The drying is controlled by the temperature of the gas and the distance the particles travel in contact with the gas. It is generally desirable to adjust these parameters to avoid too rapid drying as this results in a tendency to form dried skins on the partially dried particles of the solid phase which are subsequently ruptured as water occluded within the particles vaporizes and attempts to escape. At the same time, it is desirable to provide the catalyst in a form having as little occluded water as possible. Therefore, where a fluidized bed reactor is to be used and microspheroidal particles are desired, it is advisable to choose the conditions of spray-drying with a view to achieving substantial complete drying without particle rupture.

Following the drying operation, the catalyst precursor is calcined to form the catalyst. The calcination process is usually conducted in air at essentially atmospheric pressure and at a temperature of about 500°C to about 850°C, such as from about 600°C to about 800°C. The time to complete the calcination can be anything up to 10 hours, but for most purposes the calcination need take only from about 1 hour to about 3 hours.

In some applications, it may be advantageous to include in the catalyst a support material which functions by providing a large surface area for the catalyst and by creating a harder and more durable catalyst for use in the highly abrasive environment of a fluidized bed reactor. This support material can be any of those commonly proposed for such use, much as, for example, silica, zirconia, alumina, titania, antimony pentoxide sol, or other oxide substrates. From the point of view of availability, cost, and performance, silica is usually a satisfactory support material and is preferably in the form of silica sol for easy dispersion.

The proportions in which the components of the supported catalysts are present can vary widely, but it is usually preferred that the support provides from about 10% to about 90% and more preferably about 35% to about 65% by weight of the total combined weight of the catalyst and the support. To incorporate a support into the catalyst, the support material is preferably added to the slurry containing the active components previously discussed.

As previously stated, catalysts according to this invention are those comprising the following elements in the atomic ratio indicated:

$$Fe_aSb_bCu_cTe_dMo_eW_fMe_gO_x$$

where Me is a member of the group consisting of V, B, P, Mg, Sn, Zn, Mn, and mixtures thereof wherein a is 5 to 10, b is 10 to 30, c is 1 to 5, d is 0.55 to 5, e is 0.1 to 5, f is not greater than 5, g is 0 to 0.5, and x is taken to satisfy the valence requirements of the other elements present. In more preferred embodiments of such catalysts, a is 5 to 8, b is 10 to 20, c is 2 to 4, d is 0.5 to 3, e is 0.5 to 2, f is 0.05 to 2, and g is 0.01 to 0.2.

The catalyst preparation of this invention yields a catalyst that is useful in the production of nitriles from olefins. Olefins suitable for use in this invention include those characterized by having at least one methyl group attached to a trigonal carbon atom. Nonlimiting representatives of such olefins include propylene, isobutylene, 2-methyl-1-pentene, 1,4-hexadiene, and the like. Of particular importance is the production of acrylonitrile from propylene and in the discussion which follows, specific reference is made to that process although it should be understood that the described catalyst is also useful for ammoxidation of other suitable olefins and for oxidation of such olefins to aldehydes and acids.

In the most frequently used ammoxidation processes, a mixture of olefin, ammonia, and oxygen (or air) is fed into a reactor and through a bed of catalyst particles at elevated temperatures. Such temperatures are usually in the range of about 400°C to about 550°C, and the pressure is from about 1 atmosphere to about 6 atmospheres (100 kPa to about 600 kPa). The ammonia and olefin are required stoichiometrically in equimolar amounts, but it is usually necessary to operate with a molar ratio of ammonia to olefin in excess of 1 to reduce the incidence of side reactions. Likewise, the stoichiometric oxygen requirement is 1.5 times the molar amount of olefin. The feed mixture is commonly introduced into the catalyst bed at a W/F (defined as the weight of the catalyst in grams divided by the flow of reactant stream in ml/sec. at standard temperature and pressure) in the range of about 2 to about 15, preferably from about 4 to about 10.

The ammoxidation reaction is exothermic and for convenience in heat distribution and removal, the catalyst bed is desirably fluidized. However, fixed catalyst beds may also be employed with alternative heat removal means such as cooling coils within the bed.

The catalyst as prepared by the process of this invention is particularly well adapted for use in such a process in that the loss of tellurium exhibited by similar prior art catalysts is substantially reduced or eliminated altogether during extended use due to the unique and novel preparation procedures employed herein.

The following examples illustrating the best presently-known methods of practicing this invention are described in order to facilitate a clear understanding of the invention. It should be understood, however, that the expositions of the application of the invention, while indicating preferred embodiments, are given by way of illustration only and are not to be construed as limiting the invention since various changes will become apparent to those skilled in the art from this description.

Examples

In the examples that are presented below, the general preparation of catalyst compositions are described. These compositions should have properties beneficial for use in the ammoxidation of propylene to produce acrylonitrile.

As used herein, the following terms are defined in the following manner:

1. "W/F" is defined as the weight of the catalyst in grams divided by the flow rate of reactant stream in ml/sec. measured at STP.

2. "Propylene ($C_3H_6$) conversion" is defined as:

$$\frac{\text{mols } C_3H_6 \text{ in feed} - \text{mols } C_3H_6 \text{ in effluent}}{\text{mols } C_3H_6 \text{ in feed}} \times 100$$

3. "Acrylonitrile (AN) selectivity" is defined as:

$$\frac{\text{mols AN in effluent}}{\text{mols } C_3H_6 \text{ converted}} \times 100$$

4. "Acrylonitrile (AN) yield" is defined as:

$$\frac{\text{mols AN formed}}{\text{mols } C_3H_6 \text{ feed}} \times 100$$

In the following paragraphs, the catalysts of the examples (approximately 30 g), upon evaluation in a fluidized bed reaction vessel having an inside diameter of about 13 mm, should demonstrate high acrylonitrile selectivity and yield and propylene conversion. Pass a reactant mixture of 17—17.8 volume percent $O_2$, 7.6—8.3 volume percent propylene ($C_3H_6$), 8—9 volume percent $NH_3$, and the balance helium upward through the catalyst bed at a rate sufficient to give the value of W/F desired. Maintain the temperature between about 425°C and about 500°C (preferred temperatures) and the pressure at about 205 kPa.

Thus, it is apparent that there has been provided in accordance with the present invention, catalysts and a process for using same that fully satisfy the objects and advantages set forth hereinabove. While the invention has been described with respect to various specific examples and embodiments thereof, it is understood that the invention is not limited thereto and that many alternatives, modifications, and variations will be apparent to those skilled in the art in light of the foregoing description. Accordingly, it is intended to embrace all such alternatives, modifications, and variations as fall within the scope of the claims

Example 1 (Comparative)

A catalyst was prepared using the following ingredients in the amounts indicated:

| | |
|---|---|
| $Sb_2O_3$ | 223.4 g |
| $Fe(NO_3)_39H_2O$ | 310.3 g |
| $(NH_4)_6Mo_7O_{24}4H_2O$ | 9.2 g |
| $H_2TeO_42H_2O$ | 24.0 g |
| $Cu(NO_3)_23H_2O$ | 59.3 g |
| $NH_4VO_3$ | 1.376 g |
| $(NH_4)_6W_7O_{24}6H_2O$ | 1.62 g |
| Silica sol (Nalco 2327) | 600.0 g |

The method of preparation was as follows:

1. Dissolve $Fe(NO_3)_39H_2O$ in 190 ml of water in a 2,000 ml beaker.
2. Dissolve $(NH_4)_6Mo_7O_{24}4H_2O$ and $H_2TeO_4$ in 175 ml of water.
3. Dissolve $Cu(NO_3)_23H_2O$ in 300 ml of water.
4. Dissolve $NH_4VO_3$ and $(NH_4)_6W_7O_{24}6H_2O$ in 50 ml of water. The solution was heated to complete the dissolution.
5. Add (2), (3), and (4) in sequence to (1).
6. Add $Sb_2O_3$ and silica sol.
7. Adjust pH of the mixture to 2 with 330 ml of 1:1 $NH_4OH$ in water.
8. Heat to concentrate the slurry to a volume of 1400 ml and spray dry.
9. Calcine at 700°C for 4 hours.

Example 2

Tellurium molybdate was prepared using the following ingredients:

| | |
|---|---|
| TeO | 86.15 g |
| $MoO_3$ | 38.85 g |

Preparation of the compound was as follows:

1. Mix the oxides with 50 ml of ethanol.
2. Ball mill the mixture in a small jar overnight.
3. Evaporate at low heat the mixture to dryness and dry in an oven at 110°C for 4 hours.
4. Calcine at 500°C for 1 hour with air passing through the furnace.
5. Cool and grind the solid slightly to powder.
6. Calcine again at 500°C for another 3 hours.

A catalyst was prepared employing the above prepared tellurium molybdate with the other compounds indicated as follows:

| | |
|---|---|
| $Sb_2O_3$ | 223.4 g |
| $Fe(NO_3)_39H_2O$ | 310.3 g |
| $Cu(NO_3)_23H_2O$ | 59.3 g |
| Silica sol (Nalco 2327) | 600.0 g |
| $NH_4VO_3$ | 1.376 g |
| $(NH_4)_6W_7O_{24}6H_2O$ | 1.62 g |
| $Te_2MoO_7$ | 24.2 g |

Preparation of the catalysts was according to the following procedure:

1. Add $Sb_2O_3$ to a mixture of 323.4 ml $HNO_3$ in 223 ml of water. Heat at 95°C for 4 hours. The mixture was then diluted with 1500 ml of water and decanted twice.
2. Add Fe(NO ) 9H O dissolved in 290 ml of water.
3. Add Cu(NO ) 3H O dissolved in 150 ml of water.
4. Add silica sol. Adjust pH to 2 with 360 ml of NH OH (1:1).
5. Dissolve $NH_4VO_3$ and $(NH_4)_6W_7O_{24}6H_2O$ in 50 ml of water. Heat to complete the dissolution. Add (5) to (4).
6. Add $Te_2MoO_7$.
7. Ball mill over night (16 hours), pH 1.5. Adjust to 4 with 100 ml of $NH_4OH$.
8. Concentrate to 1600 ml, pH 3.85.
9. Spray dry.
10. Calcine at 750°C for 4 hours.

The catalysts of Examples 1 and 2 were tested in the same manner in a 13 mm diameter reactor. Reaction conditions and a description of the feed and product streams are shown in the following Table.

TABLE 1

| Preparation procedure | Example 1 | | Example 2 | |
|---|---|---|---|---|
| Temperature, °C | 425 | 430 | 425 | 430 |
| Pressure, kPa | 191 | 219 | 191 | 219 |
| Feed, %, $C_3H_6$ | 6.78 | 8.0 | 6.78 | 8.0 |
| $NH_3$ | 7.14 | 8.4 | 7.14 | 8.4 |
| $O_2$ | 18.06 | 17.5 | 18.06 | 17.5 |
| He | 68.02 | 66.1 | 68.02 | 66.1 |
| W/F, g sec $ml^{-1}$ | 5 | 6 | 5 | 6 |
| $C_3H_6$ Conv. % | 94.2 | 96.3 | 94.0 | 96.7 |
| AN Selec. % | 76.9 | 75.7 | 80.5 | 77.5 |
| AN Yield, % | 72.5 | 72.8 | 75.7 | 74.9 |
| Carbon Balance, % | 98.6 | 97.9 | 97.4 | 98.2 |

From these results, it can be seen that use of a preformed tellurium molybdate produces a catalyst with significantly enhanced acrylonitrile selectivity. The activity of the catalyst, measured by propylene conversion, is about the same as the prior art catalyst, while the AN selectivity, and consequently the AN yield, are significantly increased. In the case of operation at relatively low pressure, the AN yield increased from 72.5% to 75.7%.

Although the laboratory test lasted only 23 hours, it is believed that the tellurium molybdate catalyst will continue to remain active for extended periods of time, because tellurium molybdate is a stable component and will not lose tellurium to the same extent of the prior art catalyst. These data prove that this catalyst is substantially more selective for acrylonitrile than the prior art catalyst.

**Claims**

1. A process for the production of a mixed oxide catalyst for oxidation and ammoxidation of olefins comprising:

$$Fe_aSb_bCu_cTe_dMo_eW_fMe_gO_x$$

where Me is a member of the group consisting of V, B, P, Mg, Sn, Zn, Mn, and mixtures thereof wherein the subscripts are atomic ratios, wherein a is 5 to 10, b is 10 to 30, c is 1 to 5, d is 0.5 to 5, e is 0.1 to 5, f is not greater than 5, g is 0 to 0.5, and x is a number taken to satisfy the valence requirements of the other elements present, the process comprising:

(1) forming a mixture containing iron, antimony and copper components and a preformed tellurium-containing component selected from the group consisting of tellurium. molybdate, tellurium tungstate, and mixtures thereof, and a complementary metal-containing component, the metal constituent of which is selected from the group consisting of molybdenum, tungsten, and mixtures thereof, with the proviso that when the preformed tellurium-containing component is tellurium molybdate, the metal constituent of the complementary metal-containing component is tungsten and when the preformed tellurium-containing component is tellurium tungstate, the complementary metal constituent of the metal-containing component is molybdenum, and, optionally, vanadium and tungsten components in amounts sufficient to satisfy the elemental atomic ratio requirements,

(2) forming the mixture into dry particles,

(3) drying the particles, and

(4) calcining the particles.

2. A process of Claim 1 wherein the tellurium-containing component is tellurium molybdate and the metal-containing component is tungsten trioxide.

3. A process of either Claim 1 or Claim 2, wherein the vanadium component is vanadium antimonate.

4. A process of any of Claims 1 to 3, wherein a is 5 to 8, b is 10 to 20, c is 2 to 4, d is 0.5 to 3, e is 0.5 to 2, f is 0.05 to 2, and g is 0.01 to 0.2.

5. A process of any of Claims 1 to 4, wherein the antimony component is antimony trioxide.

6. A process of any of Claims 1 to 5, wherein the iron component is ferric nitrate.

7. A process of any of Claims 1 to 6, wherein the copper component is cupric nitrate.

8. A process of any of Claims 1 to 7, wherein the mixture is in the form of an aqueous slurry.

9. A process of Claim 8, wherein a support material comprising from 10% to 90% of the total weight of the catalyst is added to the slurry.

10. A process of Claim 9, wherein the support material comprises from 35% to 65% of the total weight of the catalyst.

11. A process of either Claim 9 or Claim 10, wherein the support material is silica.

12. A process of any of Claims 8 to 11, wherein the dry particles are formed by spray-drying the aqueous slurry.

13. A process of any of Claims 1 to 12, wherein the dry particles are calcined at a temperature from 500°C to 850°C.

14. A process of Claim 13, wherein the calcination temperature is from 600°C to 800°C.

15. A process for the production of acrylonitrile comprising reacting in the vapor phase propylene, ammonia and oxygen in the presence of a catalyst produced by a process according to any of Claims 1 to 14.

16. A process of Claim 15, wherein the reaction is conducted at a pressure of 100 kPa to 600 kPa and a temperature of 400°C to 550°C.

17. A process of Claim 16, wherein the temperature is from 425°C to 500°C.

18. A process of any of Claims 15 to 17, wherein the flow rate W/F is in the range of 2 g-sec./ml to 15 g-sec./ml.

19. A mixed oxide catalyst for oxidation and ammoxidation of olefins comprising:

$$Fe_aSb_bCu_cTe_dMo_eW_fMe_gO_x$$

where Me is a member of the group consisting of V, B, P, Mg, Sn, Zn, Mn and mixtures thereof wherein the subscripts are atomic ratios, wherein a is 5 to 10, b is 10 to 30, c is 1 to 5, d is 0.5 to 5, e is 0.1 to 5, f is not greater than 5, g is 0 to 0.5, and x is a number taken to satisfy the valence requirement of the other elements present, the catalyst being obtainable by forming a mixture containing iron, antimony and copper components, a preformed tellurium-containing component selected from the group consisting of tellurium molybdate, tellurium tungstate, and mixtures thereof, and a complementary metal-containing component, the metal constituent of which is selected from the group consisting of molybdenum, tungsten, and mixtures thereof, with the proviso that when the preformed tellurium-containing component is tellurium molybdate, the metal constituent of the complementary metal-containing component is tungsten and when the preformed tellurium-containing component is tellurium tungstate, the metal constituent of the complementary metal-containing component is molybdenum, and optionally, vanadium and tungsten

components, in amounts sufficient to satisfy the elemental atomic ratio requirements, forming the mixture into particles, and drying and calcining the particles.

20. A process for the production of acrylonitrile comprising reacting in the vapor phase propylene, ammonia and oxygen in the presence of a catalyst according to Claim 19.

**Patentansprüche**

1. Verfahren zur Herstellung eines Mischoxid-Katalysators für die Oxidation und Ammoxidation von Olefinen enthaltend

$$Fe_aSb_bCu_cTe_dMo_eW_fMe_gO_x$$

wo Me ein Element aus der V, B, P, Mg, Sn, Zn, Mn und Gemische davon umfassenden Gruppe darstellt, worin die tiefgestellten Indices die Atomverhältnisse darstellen und a 5 bis 10, b 10 bis 30, c 1 bis 5, d 0,5 bis 5, e 0,1 bis 5, f nicht größer als 5, g 0 bis 0,5 und x eine zur Erfüllung der Valenzerfordernisse der anderen vorhandenen Elemente angenommene Zahl bedeuten, durch

(1) Bilden einer Mischung, die Eisen-, Antimon- und Kupferbestandteile und einen vorgeformten Tellur enthaltenden Bestandteil, der aus der Tellurmolybdat, Tellurwolframat und Gemische davon umfassenden Gruppe ausgewählt ist, und einem ergänzenden Metall enthaltenden Bestandteil, dessen Metallanteil aus der Molybdän, Wolfram und Gemische davon umfassenden Gruppe ausgewählt ist, mit der Maßgabe enthalten, daß, wenn der vorgeformte Tellur enthaltende Bestandteil Tellurmolybdat ist, der Metallanteil des ergänzenden Metall enthaltenden Bestandteils Wolfram ist und, wenn der vorgeformte Tellur enthaltende Bestandteil Tellurwolframat ist, der Metallanteil des ergänzenden Metall enthaltenden Bestandteils Molybdän ist, und gegebenenfalls Vanadium- und Wolfram-Bestandteile in zur Erfüllung der elementaren Atomverhältnis-Erfordernisse, ausreichenden Mengen vorhanden sind.

(2) Verformen des Gemischs zu trockenen Teilchen,

(3) Trocknen der Teilchen und

(4) Calcinieren der Teilchen.

2. Verfahren nach Anspruch 1, worin der Tellur enthaltende Bestandteil Tellurmolybdat und der Metall enthaltende Bestandteil Wolframtrioxid sind.

3. Verfahren nach Anspruch 1 oder Anspruch 2, worin der Vanadiumbestandteil Vanadiumantimonat ist.

4. Verfahren nach einem der Ansprüche 1 bis 3, worin a 5 bis 8, b 10 bis 20, c 2 bis 4, d 0,5 bis 3, e 0,5 bis 2, f 0,05 bis 2, und g 0,01 bis 0,2 bedeuten.

5. Verfahren nach einem der Ansprüche 1 bis 4, worin der Antimonbestandteil Antimontrioxid ist.

6. Verfahren nach einem der Ansprüche 1 bis 5, worin der Eisenbestandteil Eisen(III)-nitrat ist.

7. Verfahren nach einem der Ansprüche 1 bis 6, worin der Kupferbestandteil Kupfer(II)-nitrat ist.

8. Verfahren nach einem der Ansprüche 1 bis 7, worin das Gemisch in Form einer wässrigen Aufschlämmung vorliegt.

9. Verfahren nach Anspruch 8, worin ein Trägermaterial, das 10% bis 90% des Gesamtgewichts des Katalysators ausmacht, zu der Aufschlämmung zugegeben wird.

10. Verfahren nach Anspruch 9, worin das Trägermaterial 35% bis 65% des Gesamtgewichts des Katalysators ausmacht.

11. Verfahren nach Anspruch 9 oder Anspruch 10, worin das Trägermaterial Siliciumdioxid ist.

12. Verfahren nach einem der Ansprüche 8 bis 11, worin die trockenen Teilchen durch Sprühtrocknen der wässrigen Aufschlämmung geformt werden.

13. Verfahren nach einem der Ansprüche 1 bis 12, worin die trockenen Teilchen bei einer Temperatur von 500°C bis 850°C calciniert werden.

14. Verfahren nach Anspruch 13, worin die Calcinierungstemperatur 600°C bis 800°C beträgt.

15. Verfahren zur Herstellung von Acrylnitril durch Umsetzen von Propylen, Ammoniak und Sauerstoff in der Dampfphase in der Gegenwart eines Katalysators, hergestellt nach einem Verfahren gemäß einem der Ansprüche 1 bis 14.

16. Verfahren nach Anspruch 15, worin das Umsetzen bei einem Druck von 100 kPa bis 600 kPa und einer Temperatur von 400°C bis 550°C durchgeführt wird.

17. Verfahren nach Anspruch 16, worin die Temperatur 425°C bis 500°C beträgt.

18. Verfahren nach einem der Ansprüche 15 bis 17, worin die Flußrate W/F in dem Bereich von 2 g-sec./ml bis 15 g-sec./ml liegt.

19. Mischoxid-Katalysator zur Oxidation und Ammoxidation von Olefinen enthaltend

$$Fe_aSb_bCu_cTe_dMo_eW_fMe_gO_x$$

wo Me ein Element aus der V, B, P, Mg, Sn, Zn, Mn und Gemische davon umfassenden Gruppe darstellt, worin die tiefgestellten Indices die Atomverhältnisse darstellen und a 5 bis 10, b 10 bis 30, c 1 bis 5, d 0,5 bis 5, e 0,1 bis 5, f nicht größer als 5, g 0 bis 0,5 und x eine zur Erfüllung der Valenzerfordernisse der anderen vorhandenen Elemente angenommene Zahl bedeuten, wobei der Katalysator durch Bilden einer Mischung,

die Eisen-, Antimon- und Kupferbestandteile, einen vorgeformten Tellur enthaltenden Bestandteil, der aus der Tellurmolybdat, Tellurwolframat und Gemische davon umfassenden Gruppe ausgewählt ist, und einen ergänzenden Metall enthaltenden Bestandteil, dessen Metall-anteil aus der Molybdän, Wolfram und Gemische davon umfassenden Gruppe ausgewählt ist, mit der Maßgabe enthält, daß, wenn der vorgeformte Tellur enthaltende Bestandteil Tellurmolybdat ist, der Metallanteil des ergänzenden Metall enthaltenden Bestandteils Wolfram ist, und wenn der vorgeformte Tellur enthaltende Bestandteil Tellurwolframat ist, der Metallanteil des ergänzenden Metall enthaltenden Bestandteils Molybdän ist, und, gegebenenfalls Vanadium- und Wolframbestandteile in zur Erfüllung der elementaren Atomverhältnis-Erfordernisse ausreichenden Mengen vorhanden sind, Verformen des Gemischs zu Teilchen, und Trocknen und Calcinieren der Teilchen erhältlich ist.

20. Verfahren zur Herstellung von Acrylnitril durch Umsetzen von Propylen, Ammoniak und Sauerstoff in der Dampfphase in der Gegenwart eines Katalysators nach Anspruch 19.

**Revendications**

1. Procédé pour la production d'un catalyseur d'oxyde mixte pour l'oxydation et l'ammoxydation d'oléfines comprenant:

$$Fe_aSb_bCu_cTe_dMo_eW_fMe_gO_x$$

formule dans laquelle Me est un élément du groupe constitué par V, B, P, Mg, Sn, Zn, Mn, et leurs mélanges, sachant que les indices représentent les rapports atomiques, a est compris entre 5 et 10, b est compris entre 10 et 30, c est compris entre 1 et 5, d est compris entre 0,5 et 5, e est compris entre 0,1 et 5, f n'est pas supérieur à 5, g est compris entre 0 et 0,5 et x est un nombre permettant de satisfaire aux exigences en ce qui concerne la valence des autres éléments présents, le procédé comprenant:

(1) la formation d'un mélange contenant des composants fer, antimoine et cuivre et un composant contenant du tellure pré-formé choisi dans le groupe constitué par du molybdate de tellure, du tungstate de tellure et leurs mélanges et un composant contenant un métal complémentaire dont le constituant métallique est choisi dans le groupe constitué par le molybdène, le tungstène et leurs mélanges, à condition que lorsque le composant contenant du tellure pré-formé est du molybdate de tellure, le constituant métallique du composant contenant un métal complémentaire soit le tungstène et lorsque le composant contenant du tellure pré-formé est du tungstate de tellure, le constituant métallique complémentaire du composant contenant un métal soit le molybdène et éventuellement des composants vanadium et tungstène en des quantités suffisantes pour satisfaire aux exigences en ce qui concerne les rapports atomiques élémentaires,

(2) la mise du mélange sous forme de particules sèches,

(3) le séchage des particules et

(4) la calcination des particules.

2. Procédé selon la revendication 1, dans lequel le composant contenant du tellure est du molybdate de tellure et le composant contenant un métal est du trioxyde de tungstène.

3. Procédé selon la revendication 1 ou la revendication 2, dans lequel le composant vanadium est de l'antimoniate de vanadium.

4. Procédé selon l'une quelconque des revendications 1 à 3, dans lequel a est compris entre 5 et 8, b est compris entre 10 et 20, c est compris entre 2 et 4, d est compris entre 0,5 et 3, est compris entre 0,5 et 2, f est compris entre 0,05 et 2 et g est compris entre 0,01 et 0,2.

5. Procédé selon l'une quelconque des revendications 1 à 4, dans lequel le composant antimoine est le trioxyde d'antimoine.

6. Procédé selon l'une quelconque des revendications 1 à 5, dans lequel le composant fer est le nitrate ferrique.

7. Procédé selon l'une quelconque des revendications 1 à 6, dans lequel le composant cuivre est le nitrate cuivrique.

8. Procédé selon l'une quelconque des revendications 1 à 7, dans lequel le mélange se trouve sous la forme d'une bouillie aqueuse.

9. Procédé selon la revendication 8, dans lequel on ajoute un matériau support représentant de 10% à 90% du poids total du catalyseur à la bouillie.

10. Procédé selon la revendication 9, dans lequel le matériau support représente de 35% à 65% du poids total du catalyseur.

11. Procédé selon la revendication 9 ou la revendication 10, dans lequel le matériau support est de la silice.

12. Procédé selon l'une quelconque des revendications 8 à 11, dans lequel on forme les particules sèches en séchant par pulvérisation la bouillie aqueuse.

13. Procédé selon l'une quelconque des revendications 1 à 12, dans lequel on calcine les particules sèches à une température comprise entre 500°C et 850°C.

14. Procédé selon la revendication 13, dans lequel la température de calcination est comprise entre 600°C et 800°C.

15. Procédé pour la production d'acrylonitrile, comprenant la mise en réaction en phase vapeur de propylène, d'ammoniac et d'oxygène en présence d'un catalyseur produit par un procédé selon l'une quelconque des revendications 1 à 14.

16. Procédé selon la revendication 15, dans lequel on met en oeuvre la réaction sous une pression de 100 kPa à 600 kPa et à une température de 400°C à 550°C.

17. Procédé selon la revendication 16, dans lequel la température est comprise entre 425°C et 500°C.

18. Procédé selon l'une quelconque des revendications 15 à 17, dans lequel le débit d'écoulement W/F est compris entre 2 g-s/ml et 15 g-s/ml.

19. Catalyseur d'oxyde mixte pour l'oxydation et l'ammoxydation d'oléfines comprenant:

$$Fe_aSb_bCu_cTe_dMo_eW_fMe_gO_x$$

formule dans laquelle Me est un élément du groupe constitué par V, B, P, Mg, Sn, Zn, Mn et leurs mélanges, sachant que les indices représentent les rapports atomiques, a est compris entre 5 et 10, b est compris entre 10 et 30, c est compris entre 1 et 5, d est compris entre 0,5 et 5, e est compris entre 0,1 et 5, f n'est pas supérieur à 5, g est compris entre 0 et 0,5 et x est un nombre permettant d satisfaire aux exigences en ce qui concerne la valence des autres éléments présents, sachant que l'on peut obtenir le catalyseur en formant un mélange contenant des composants fer, antimoine et cuivre, un composant contenant du tellure pré-formé choisi dans le groupe constitué par du molybdate de tellure, du tungstate de tellure et leurs mélanges et un composant contenant un métal complémentaire dont le constituant métallique est choisi dans le groupe constitué par le molybdène, le tungstène et leurs mélanges, à condition que lorsque le composant contenant du tellure pré-formé est du molybdate de tellure, le constituant métallique du composant contenant un métal complémentaire soit le tungstène et lorsque le composant contenant du tellure pré-formé est du tungstate de tellure, le constituant métallique du composant contenant un métal complémentaire soit le molybdène et éventuellement des composants vanadium et tungstène, en des quantités suffisantes pour satisfaire aux exigences en ce qui concerne les rapports atomiques élémentaires, en mettant le mélange sous forme de particules et en séchant et en calcinant les particules.

20. Procédé pour la production d'acrylonitrile comprenant la mise en réaction en phase vapeur de propylène, d'ammoniac et d'oxygène en présence d'un catalyseur selon la revendication 19.